(19) **Europäisches Patentamt**
**European Patent Office**
**Office européen des brevets**

(11) **EP 4 375 898 B1**

(12) **EUROPEAN PATENT SPECIFICATION**

(45) Date of publication and mention
of the grant of the patent:
**31.12.2025  Bulletin 2026/01**

(21) Application number: **23211932.1**

(22) Date of filing: **24.11.2023**

(51) International Patent Classification (IPC):
***G06Q 10/06*** *(2023.01)*

(52) Cooperative Patent Classification (CPC):
**G06Q 10/06**

(54) **APPARATUS, METHOD AND COMPUTER-READABLE STORAGE MEDIUM FOR ALLOCATING OPERATING ROOMS BASED ON UTILIZATION RATE BY TIME PERIOD**

VORRICHTUNG, VERFAHREN UND COMPUTERLESBARES SPEICHERMEDIUM ZUR ZUWEISUNG VON BETRIEBSRÄUMEN JE NACH NUTZUNGSRATE NACH ZEITPERIODE

APPAREIL, PROCÉDÉ ET SUPPORT DE STOCKAGE LISIBLE PAR ORDINATEUR POUR ATTRIBUER DES SALLES D'OPÉRATION SUR LA BASE D'UN TAUX D'UTILISATION PAR PÉRIODE DE TEMPS

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority:  **25.11.2022  KR 20220160924**

(43) Date of publication of application:
**29.05.2024  Bulletin 2024/22**

(73) Proprietor: **Samsung Life Public Welfare Foundation**
**Seoul 04348 (KR)**

(72) Inventors:
  • **MOON, Jung Hwan**
    **06351 Seoul (KR)**
  • **WOO, Ji Hye**
    **06351 Seoul (KR)**
  • **CHOI, Jun Young**
    **06351 Seoul (KR)**
  • **SEOL, Ho Jun**
    **06351 Seoul (KR)**
  • **CHOI, Jae Kyun**
    **06351 Seoul (KR)**
  • **JEONG, Seok Doo**
    **06351 Seoul (KR)**
  • **PARK, Chae Yeon**
    **06351 Seoul (KR)**
  • **JU, Mi Ja**
    **06351 Seoul (KR)**
  • **Lee, Ihn Seon**
    **06351 Seoul (KR)**
  • **Lim, Do Hoon**
    **06351 Seoul (KR)**

(74) Representative: **Weickmann & Weickmann PartmbB**
**Postfach 860 820**
**81635 München (DE)**

(56) References cited:
    **CA-A1- 3 161 894      US-A- 4 075 679**
    **US-B1- 11 341 462**

## Description

BACKGROUND

1. Field

One or more embodiments relate to an apparatus, method, and computer-readable recording medium for allocating operating rooms based on a utilization rate by time period.

2. Description of the Related Art

As medical technology advances, customers' medical options are expanding and medical services are becoming more customer-centered, there is a need for efficient operation of operating rooms owned by hospitals so that customers may conveniently and quickly receive treatment at hospitals.

In particular, the higher the ratio of non-operating time compared to operating time when an operating room is actually used, the longer customers have to wait for surgery, and the hospital cannot generate profits efficiently, making it difficult to provide good service.

In addition, when a utilization rate of each operating room owned by a hospital varies greatly, working hours of each operating room in charge of the surgeon may vary, which may result in the surgeon working overtime in a specific operating room even though there is an empty operating room. Therefore, there may be a waste of human and material resources.

Accordingly, a plan is needed to efficiently allocate operating rooms so that utilization rates of operating rooms may be adjusted to an appropriate range and the variation in operating room utilization rates may be reduced.

Document CA 3 161 894 A1 discloses optimizing patient placement and sequencing in a dynamic medical system. Document US 11 341 462 B1 relates to generating and/ or updating a schedule, the schedule including a recurring appointment report based upon user input.

SUMMARY

One or more embodiments include a technology that introduces the concept of an operation rate by time period to calculate an operation rate of each operating room by time period, and based on an average operation rate of each operating room by time period for a period set by a user, adjusts an operation rate of an operating room to an appropriate range and allocates operating rooms so that the deviation in operation rate by operating room is reduced.

TECHNICAL SOLUTION

As an embodiment of the present disclosure, an operating room allocation apparatus may be provided.

The apparatus according to an embodiment of the present disclosure may include: an information obtaining unit configured to obtain information about patient's entry and exit times in each operating room; a first calculation unit configured to calculate a utilization rate of the operating room for each certain time period based on the patient's entry and exit times; and a second calculation unit configured to receive certain time information from a user and calculate an average utilization rate by time period in the received time information.

The operating room allocation apparatus according to an embodiment of the present disclosure, wherein the information obtaining unit may further obtain information about downtime of the operating room, and the first calculation unit specify a time obtained by adding the downtime to the exit time as a final exit time, may calculate a time from the entry time to the final exit time as a time the operating room was used, and calculate a utilization rate of the operating room for the each certain time period.

The operating room allocation apparatus according to an embodiment of the present disclosure, wherein the first calculation unit may divide a time from time information specified as the entry time to time information specified as the exit time by preset time intervals, may calculate a ratio of the operating room used for each of the preset time intervals, and calculate a utilization rate of the operating room.

The operating room allocation apparatus according to an embodiment of the present disclosure, wherein, when the entry time is x1:y1, the exit time is x2:y2, x1 = x2, and the certain time is set at 30-minute intervals, the first calculation unit may calculate a utilization rate from x1:00 to x1:30 as $\frac{(y2 - y1)}{30}$ when minute information y1 of the entry time and minute information y2 of the exit time are 30 minutes before or 30 minutes, may calculate a utilization rate from x1:30 to x1:60 as $\frac{(y2 - y1)}{30}$ when minute information y1 of the entry time and minute information y2 of the exit time are 30 minutes after, and may calculate a utilization rate from x1:00 to x1:30 as $\frac{(30 - y1)}{30}$ and calculates a utilization rate from x1:30 to x1:60 as $\frac{(y2-30)}{30}$ when minute information y1 of the entry time is 30 minutes before or 30 minutes, and minute information y2 of the exit time is 30 minutes after.

The operating room allocation apparatus according to an embodiment of the present disclosure, wherein, when the entry time is x1:y1, the exit time is x2:y2, x1 ≠ x2, and the certain time is set at 30-minute intervals, the first calculation unit may calculate a utilization rate from x1:00 to x1:30 as $\frac{(30 - y1)}{30}$ and calculates

a utilization rate from x1:30 to x1:60 as 1 when minute information y1 of the entry time is 30 minutes before or 30 minutes, may calculate a utilization rate from x1:30 to x1:60 as $\frac{(60-y1)}{30}$ when minute information y1 of the entry time is 30 minutes after, may calculate a utilization rate from x2:00 to x2:30 as $\frac{y2}{30}$ when minute information y2 of the exit time is 30 minutes before or 30 minutes, may calculate a utilization rate from x2:00 to x2:30 as 1 and calculates a utilization rate from x2:30 to x2:60 as $\frac{(y2-30)}{30}$ when minute information y2 of the exit time is 30 minutes after, and may calculate a utilization rate from a:00 to a:30 as 1, and a utilization rate from a:30 to a:60 as 1 for an integer 'a' that satisfies condition x1< a <x2.

The operating room allocation apparatus according to an embodiment of the present disclosure, wherein, when the entry time is x1:y1, the exit time is x2:y2, x1 ≠ x2, and the certain time is set at 30-minute intervals, the first calculation unit may calculate a utilization rate from x1:00 to x1:30 as $\frac{(30-y1)}{30}$ and calculates a utilization rate from x1:30 to x1:60 as 1 when minute information y1 of the entry time is 30 minutes before or 30 minutes, may calculate a utilization rate from x1:30 to x1:60 as $\frac{(60-y1)}{30}$ when minute information y1 of the entry time is 30 minutes after, may calculate a utilization rate from x2:00 to x2:30 as $\frac{y2}{30}$ when minute information y2 of the exit time is 30 minutes before or 30 minutes, may calculate a utilization rate from x2:00 to x2:30 as 1 and calculates a utilization rate from x2:30 to x2:60 as $\frac{(y2-30)}{30}$ when minute information y2 of the exit time is 30 minutes after, and may calculate a utilization rate from a:00 to a:30 as 1, and a utilization rate from a:30 to a:60 as 1 for an integer 'a' that satisfies condition x1< a <x2.

The operating room allocation apparatus according to an embodiment of the present disclosure, wherein, when x1 > x2, the first calculation unit may calculate a utilization rate from b:00 to b:30 as 1, and a utilization rate from b:30 to b:60 as 1 for an integer b that satisfies condition x1< b <23, and may calculate a utilization rate from c:00 to c:30 as 1, and a utilization rate from c:30 to c:60 as 1 for an integer c that satisfies condition 0< c <x2.

The operating room allocation apparatus according to an embodiment of the present disclosure, wherein, when the entry time is x1:y1, the exit time is x2:y2, x1 = x2, and the certain time is set at 1 hour intervals, the first calculation unit may calculate a utilization rate at x1 o'clock as $\frac{(y2-y1)}{60}$.

The operating room allocation apparatus according to an embodiment of the present disclosure, wherein, when the entry time is x1:y1, the exit time is x2:y2, x1 ≠ x2, and the certain time is set at 1 hour intervals, the first calculation unit may calculate a utilization rate at x1 o'clock as $\frac{(60-y1)}{60}$ and calculates a utilization rate at x2 o'clock as $\frac{y2}{60}$, and may calculate a utilization rate at 'a' o'clock as 1 for an integer 'a' that satisfies condition x1< a <x2.

The operating room allocation apparatus according to an embodiment of the present disclosure, wherein, when x1 > x2, the first calculation unit may calculate a utilization rate at b o'clock as 1 for an integer b that satisfies condition x1< b <23, and may calculate a utilization rate at c o'clock as 1 for an integer c that satisfies condition 0< c <x2.

The operating room allocation apparatus according to an embodiment of the present disclosure, wherein the second calculation unit may receive time information of at least one of day of the week, time period, and period from a user, filters a utilization rate by time period that satisfies the received time information, and then may calculate an average utilization rate by time period by averaging the filtered utilization rate by time period in the received time information.

The operating room allocation apparatus according to an embodiment of the present disclosure may further include: an operating room allocation unit configured to allocate, from among operating rooms, an operating room where a patient can be operated on based on an average utilization rate of each of the operating rooms by time period.

The operating room allocation apparatus according to an embodiment of the present disclosure, wherein the operating room allocation unit may allocate an operating room where a patient can be operated on from among the operating rooms, so that an average utilization rate of each of the operating rooms by time period falls within a certain range.

As an embodiment of the present disclosure, an operating room allocation method performed by an operating room allocation apparatus may be provided.

The method according to an embodiment of the present disclosure may include: obtaining information about patient's entry and exit times in an operating room; calculating a utilization rate of the operating room for each certain time period based on the patient's entry and exit times; and receiving certain time information from a user and calculating an average utilization rate by time period in the received time information.

The operating room allocation method according to an embodiment of the present disclosure, wherein the obtaining may include obtaining information about downtime of the operating room, and the calculating of the utilization rate of the operating room may include calculating the utilization rate of the operating room for the each certain time period by additionally considering the downtime.

The operating room allocation method according to an embodiment of the present disclosure, wherein the calculating of the average utilization rate by time period may include receiving time information of at least one of day of the week, time period, and period from a user, filtering a utilization rate by time period that satisfies the received time information, and then calculating an average utilization rate by time period by averaging the filtered utilization rate by time period in the received time information.

The operating room allocation method according to an embodiment of the present disclosure may further include allocating, from among operating rooms, an operating room where a patient can be operated on based on the average utilization rate.

The operating room allocation method according to an embodiment of the present disclosure, wherein the allocating may include: allocating an operating room where a patient can be operated on from among the operating rooms, so that an average utilization rate of each of the operating rooms by time period falls within a certain range.

As an embodiment of the present disclosure, a non-transitory computer-readable recording medium having recorded thereon a computer program may be provided.

The non-transitory computer-readable recording medium having recorded thereon instructions executable by a processor to: obtain information about patient's entry and exit times in an operating room; calculate a utilization rate of the operating room for each certain time period based on the patient's entry and exit times; and receive certain time information from a user and calculate an average utilization rate by time period in the received time information.

BRIEF DESCRIPTION OF THE DRAWINGS

FIG. 1 is a functional block diagram of an operating room allocation apparatus 100 according to an embodiment.
FIG. 2 is an exemplary view showing results of the operating room allocation apparatus 100 calculating an average utilization rate for 30-minute intervals for each of five operating rooms during '07:00 to 22:00' on Mondays in the past month.
FIG. 3 is an exemplary view showing results of the operating room allocation apparatus 100 calculating an average utilization rate per hour for each of five operating rooms during '00:00 to 24:00' on Tuesdays of the past three months.
FIG. 4 is an exemplary view showing results of the operating room allocation apparatus 100 calculating an average utilization rate for 30-minute intervals for each operating room during '07:00 to 22:00' on Wednesdays in the last 12 months and calculating a morning utilization rate and afternoon utilization rate for each operating room.
FIG. 5 is a flowchart showing operations of an operating room allocation method performed by the operating room allocation apparatus 100 according to an embodiment.

DETAILED DESCRIPTION

Hereinafter, embodiments of the present invention will be described with reference to the drawings. In the following description, descriptions of a well-known technical configuration in relation to a lead implantation system for a deep brain stimulator will be omitted. For example, descriptions of the configuration/structure/-method of a device or system commonly used in deep brain stimulation, such as the structure of an implantable pulse generator, a connection structure/method of the implantable pulse generator and a lead, and a process for transmitting and receiving electrical signals measured through the lead with an external device, will be omitted. Even if these descriptions are omitted, one of ordinary skill in the art will be able to easily understand the characteristic configuration of the present invention through the following description.

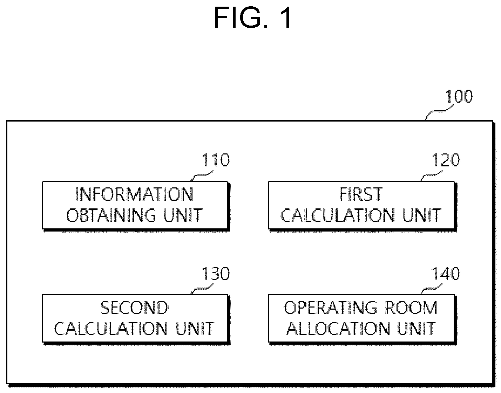

FIG. 1 is a functional block diagram of an operating room allocation apparatus 100 according to an embodiment.

The operating room allocation apparatus 100 of FIG. 1 may include an information obtaining unit 110, a first calculation unit 120, a second calculation unit 130, and an operating room allocation unit 140. The operating room allocation apparatus 100 may have all operations performed by a memory that stores data and instructions and one or more processors. One or more processors may control functional blocks included in FIG. 1 to perform operations to be described later.

The information obtaining unit 110 may obtain information about patient's entry and exit times in each operating room, or information about downtime, which is a time to prepare for surgery in the operating room. For example, the information obtaining unit 110 may operate in conjunction with an entry/exit record server or an entry/exit record database that records patient's entry time, exit time, and downtime.

The first calculation unit 120 may calculate a utilization rate of each operating room. The utilization rate refers to the percentage of time an operating room is occupied during a specific period of time (e.g., time from patient entering the operating room to leaving the operating room, time to prepare for surgery in the operating room, etc.).

To achieve a goal of more efficient operating room allocation, an embodiment of this specification introduces the concept of 'a utilization rate of an operating room by time period' to provide a more detailed understanding of how the operating room is operated at a specific time.

The first calculation unit 120 may calculate a utilization rate of an operating room by time period based on information about patient's entry and exit times in each operating room. The first calculation unit 120 may calculate a utilization rate of an operating room by time period by additionally considering information about a downtime as a time an operating room is occupied. For example, the first calculation unit 120 may specify a time obtained by adding a downtime to an exit time as a final exit time, calculate a time from an entry time to the final exit time as a time an operating room was used, and calculate a utilization rate of the operating room by time period. Standards for entry and exit times according to an embodiment of this specification may be applied in various embodiments depending on administrator's settings.

The first calculation unit 120 may divide a time from time information specified as an entry time to time information specified as an exit time by preset time intervals, calculate a ratio of an operating room used for each of the preset time intervals, and calculate a utilization rate of the operating room.

As an example, the first calculation unit 120 may calculate a utilization rate of an operating room by time period in '30 minute' units. For example, when a patient's entry time in a specific operating room is expressed as x1:y1, and a patient's exit time is x2:y2, the first calculation unit 120 may calculate a utilization rate of the operating room per 30 minutes, as shown in 1) to 10) below, according to relationships between x1, y1, x2, and y2. In more detail, when 'hours' of entry and exit times are the same or different, a calculation method may be divided as follows depending on which time period interval (e.g., 0 minutes to 30 minutes or 30 minutes to 60 minutes) 'minutes' of the entry and exit times are included.

1) When 'hours' of entry and exit times are the same, and 'minutes' of entry and exit times are within the same time period interval (e.g., 0 minutes to 30 minutes), the calculation is as follows. That is, when x1 = x2 and minute information y1 of an entry time and minute information y2 of an exit time are 30 minutes before or 30 minutes, a 30-minute utilization rate from x1:00 to x1:30 may be calculated as

$$\frac{(y2 - y1)}{30}.$$

2) When 'hours' of entry and exit times are the same, and 'minutes' of entry and exit times are within the same time period interval (e.g., 30 minutes to 60 minutes), the calculation is as follows. That is, when x1 = x2 and minute information y1 of an entry time and minute information y2 of an exit time are 30 minutes after, a 30-minute utilization rate from x1:30 to x1:60 may be calculated as $\frac{(y2 - y1)}{30}$.

3) When 'hours' of entry and exit times are the same, and 'minutes' of entry and exit times are included in different time period intervals, the calculation is as follows. That is, when x1 = x2 and minute information y1 of an entry time is 30 minutes before or 30 minutes and minute information y2 of an exit time is 30 minutes after, a 30-minute utilization rate from x1:00 to x1:30 may be calculated as $\frac{(30 - y1)}{30}$, and a 30-minute utilization rate from x1:30 to x1:60 may be calculated as $\frac{(y2 - 30)}{30}$.

4) When 'hours' of entry and exit times are different, and the entry time is 30 minutes before or 30 minutes, the calculation of a utilization rate for a time period that includes the first entry time is as follows. That is, when x1 ≠ x2 and minute information y1 of an entry time is 30 minutes before or 30 minutes, a 30-minute utilization rate from x1:00 to x1:30 may be calculated as $\frac{(30 - y1)}{30}$, and a 30-minute utilization rate from x1:30 to x1:60 may be calculated as 1.

5) When 'hours' of entry and exit times are different, and the entry time is 30 minutes after, the calculation of a utilization rate for a time period that includes the first entry time is as follows. That is, when x1 ≠ x2 and minute information y1 of an entry time is 30 minutes after, a 30-minute utilization rate from x1:30 to x1:60 may be calculated as $\frac{(60 - y1)}{30}$.

6) When 'hours' of entry and exit times are different, and the exit time is 30 minutes before or 30 minutes, the calculation of a utilization rate for a time period that includes the exit time is as follows. That is, when x1 ≠ x2 and minute information y2 of an exit time is 30 minutes before or 30 minutes, a 30-minute utilization rate from x2:00 to x2:30 may be calculated as $\frac{y2}{30}$.

7) When 'hours' of entry and exit times are different, and the exit time is 30 minutes after, the calculation of a utilization rate for a time period that includes the exit time is as follows. That is, when x1 ≠ x2 and minute information y2 of an exit time is 30 minutes after, a 30-minute utilization rate from x2:00 to x2:30 may be calculated as 1, and a 30-minute utilization rate from x2:30 to x2:60 may be calculated as

$$\frac{(y2-30)}{30}.$$

8) When 'hours' of entry and exit times are different, the calculation of a utilization rate by time period between the entry and exit times is as follows. That is, when x1 ≠ x2 and there is an integer 'a' that satisfies condition x1< a <x2, a 30-minute utilization rate from a:00 to a:30 may be calculated as 1, and a 30-minute utilization rate from a:30 to a:60 may be calculated as 1.

9) When dates of entry and exit times are different, the calculation of a utilization rate by time period corresponding to the date including the entry time is as follows. That is, when x1 > x2 and there is an integer b that satisfies condition x1< b ≤23, a 30-minute utilization rate from b:00 to b:30 may be calculated as 1, and a 30-minute utilization rate from b:30 to b:60 may be calculated as 1.

10) When dates of entry and exit times are different, the calculation of a utilization rate by time period corresponding to the date including the exit time is as follows. That is, when x1 > x2 and there is an integer c that satisfies condition 0 ≤ c <x2, a 30-minute utilization rate from c:00 to c:30 may be calculated as 1, and a 30-minute utilization rate from c:30 to c:60 may be calculated as 1.

In 1) to 10) above, the formula 'x 100', which represents a utilization rate as a percentage, may be added according to designer's settings.

As an example, the first calculation unit 120 may calculate a utilization rate of an operating room by time period in '1 hour' units. For example, when a patient's entry time in a specific operating room is expressed as x1:y1, and a patient's exit time is x2:y2, the first calculation unit 120 may calculate a utilization rate of the operating room per hour, as shown in 1) to 10) below, according to relationships between x1, y1, x2, and y2. In more detail, a calculation method may be divided as follows depending on whether 'hours' of entry and exit times are the same or different.

1) When 'hours' of entry and exit times are the same, the calculation is as follows. That is, when x1 = x2, a 1-hour utilization rate at x1 o'clock may be calculated as $\frac{(y2-y1)}{60}$.

2) When 'hours' of entry and exit times are different, the calculation is as follows. That is, when x1 ≠ x2, a 1-hour utilization rate at x1 o'clock may be calculated as $\frac{(60-y1)}{60}$, and a 1-hour utilization rate at x2 o'clock may be calculated as $\frac{y2}{60}$.

3) When 'hours' of entry and exit times are different, the calculation between the entry and exit times is as follows. That is, when there is an integer 'a' that satisfies condition x1< a <x2, a 1-hour utilization rate at 'a' o'clock may be calculated as 1.

4) When dates of entry and exit times are different, the calculation of a utilization rate by time period corresponding to the date including the entry time is as follows. That is, when x1 > x2 and there is an integer b that satisfies condition x1< b ≤23, a utilization rate at b o'clock may be calculated as 1.

5) When dates of entry and exit times are different, the calculation of a utilization rate by time period corresponding to the date including the exit time is as follows. That is, when x1 > x2 and there is an integer c that satisfies condition 0≤ c <x2, a 1-hour utilization rate at c o'clock may be calculated as 1.

In 1) to 5) above, 'x 100', which represents a utilization rate as a percentage, may be added according to designer's settings.

The second calculation unit 130 may receive certain time information from a user and calculate an average utilization rate by time period in the received time information. For example, the received time information may include at least one of a specific day of the week, a specific time period, and a specific period.

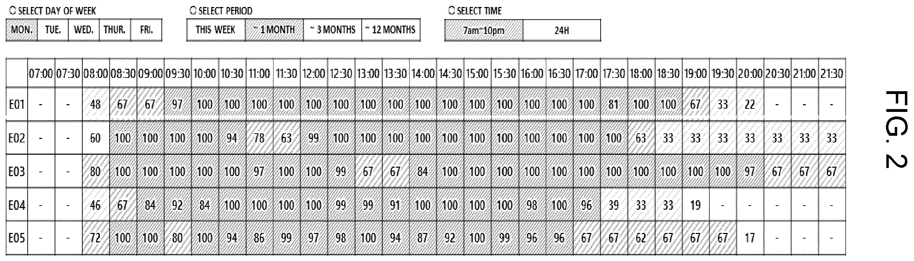

FIG. 2 is an exemplary view showing results of the operating room allocation apparatus 100 calculating an average utilization rate for 30-minute intervals for each of five operating rooms during '07:00 to 22:00' on Mondays in the past month.

Referring to FIG. 2, when a user enters 'Monday' as the day of the week, 'Last 1 month' as the period, and '07:00-22:00' as the time period, the second calculation unit 130 may filter 30-minute utilization rate information between '07:00 to 22:00' on 'Monday' in the 'last 1 month' and calculate an average utilization rate by time period in the entered time information.

For example, among filtered utilization information, when 30-minute utilization information for operating room E01 from 08:00 to 08:30 is '65, 66, 67, 68, and 69', the second calculation unit 130 may average the utilization rate information and calculate an average utilization rate from 08:00 to 08:30 in the entered time information as '67'.

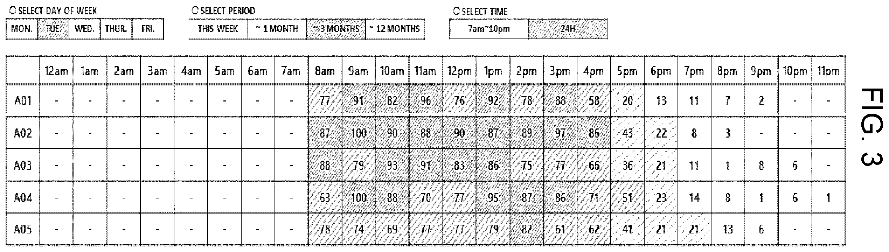

FIG. 3 is an exemplary view showing results of the operating room allocation apparatus 100 calculating an average utilization rate per hour for each of five operating rooms during '00:00 to 24:00' on Tuesdays of the past three months.

Referring to FIG. 3, when a user enters 'Tuesday' as the day of the week, 'Last 3 months' as the period, and '00:00-24:00' as the time period, the second calculation unit 130 may filter one-hour utilization rate information between '00:00 to 24:00' on 'Tuesday' of the 'last three months' and calculate an average utilization rate by time period in the entered time information.

For example, among filtered utilization information, when one-hour utilization information for operating room A01 from 08:00 to 09:00 is '73, 74, 75, 76, 77, 78, 78, 79, 80, 81, 82, and 83', the second calculation unit 130 may average the utilization rate information and calculate an average utilization rate from 08:00 to 09:00 in the entered time information as '78'.

The operating room allocation unit 140 may allocate an operating room where a patient can be operated on based on an average utilization rate of each operating room by time period. For example, the operating room allocation unit 140 may allocate an operating room where a new patient can be operated on so that an average utilization rate of each operating room by time period falls within a certain range.

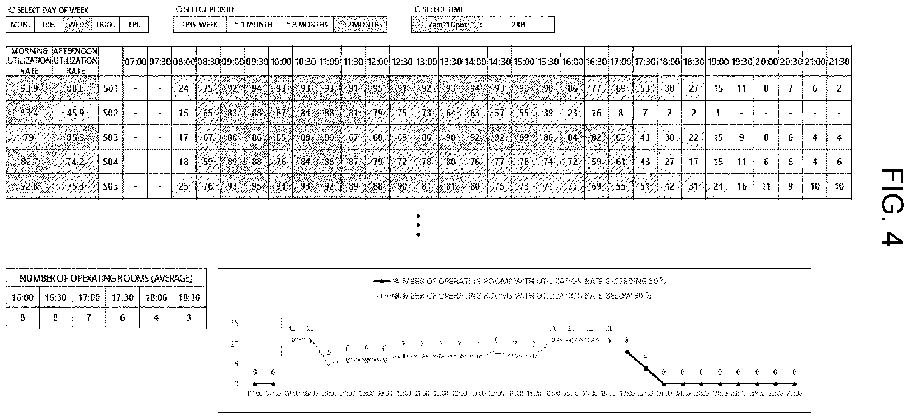

FIG. 4 is an exemplary view showing results of the operating room allocation apparatus 100 calculating an average utilization rate for 30-minute intervals for each operating room during '07:00 to 22:00' on Wednesdays in the last 12 months, and calculating a morning utilization rate and afternoon utilization rate for each operating room.

Referring to FIG. 4, when a user enters 'Wednesday' as the day of the week, 'Last 12 month' as the period, and '07:00-22:00' as the time period, the second calculation unit 130 may filter 30-minute utilization rate information between '07:00 to 22:00' on 'Wednesday' in the 'last 12 month' and calculate an average utilization rate by time period in the entered time information.

The second calculation unit 130 may calculate an average utilization rate of each operating room in a morning time period by averaging utilization rates in the morning time period (e.g., 07:00 to 12:00) based on an average utilization rate of each operating room. For example, the second calculation unit 130 may calculate the average utilization rate of a specific operating room in a morning time period by averaging average utilization rates for 30-minute intervals from 07:00 to 12:00 of the specific operating room. However, a standard for a morning time period for calculating an average utilization rate in a morning time period may vary depending on administrator's settings. For example, '08:00-12:30' may be set as a morning time period, which is the standard for calculation. In this case, an average utilization rate of a specific operating room in a morning time period may be calculated by averaging average utilization rates for 30-minute intervals from 08:00 to 12:30 for the specific operating room.

The second calculation unit 130 may calculate an average utilization rate of each operating room in an afternoon time period by averaging utilization rates in the afternoon time period (e.g., 12:00 to 22:00) based on an average utilization rate of each operating room. For example, the second calculation unit 130 may calculate the average utilization rate of a specific operating room in an afternoon time period by averaging average utilization rates for 30-minute intervals from 12:00 to 22:00 for the specific operating room. However, a standard for an afternoon time period for calculating an average utilization rate in an afternoon time period may vary depending on administrator's settings. For example, '12:30-17:00' may be set as an afternoon time period, which is a standard for calculation. In this case, an average utilization rate of a specific operating room in an afternoon time period may be calculated by averaging average utilization rates for 30-minute intervals from 12:30 to 17:30 for the specific operating room.

The operating room allocation unit 140 may allocate a specific operating room to a department or surgeon based on the average utilization rate in the morning time period or the average utilization rate in the afternoon time period.

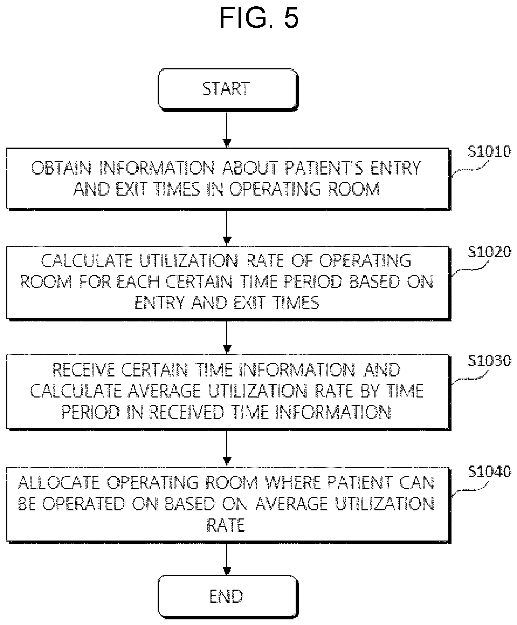

FIG. 5 is a flowchart showing operations of an operating room allocation method performed by the operating room allocation apparatus 100 according to an embodiment. Each operation of the operating room allocation method according to FIG. 5 may be performed by the operating room allocation apparatus 100 illustrated in FIG. 1, and each operation is described as follows.

In operation S1010, the information obtaining unit 110 may obtain information about patient's entry and exit times in an operating room.

In operation S1020, the first calculation unit 120 may calculate a utilization rate of the operating room for each certain time period based on the entry and exit times.

In operation S1030, the second calculation unit 130 may receive certain time information from a user and calculate an average utilization rate by time period in the received time information.

In operation S1040, the operating room allocation unit 140 may allocate an operating room where a patient can be operated on based on the average utilization rate.

In addition to the operations shown in FIG. 5, because the information obtaining unit 110, first calculation unit 120, second calculation unit 130, and operating room allocation unit 140 described above configure various embodiments to perform the operations described with FIG. 1, even in the operations of FIG. 5, a new operation performed by each functional block may be added. Because a configuration of an additional operation and operations of components responsible for each operation to perform the operation have been described in FIGS. 1 to 3, redundant descriptions will be omitted.

According to the embodiment described above, by calculating a utilization rate of each operating room by time period and using an average utilization rate of each operating room by time period for a period set by a user, an operating room utilization rate may be adjusted to an appropriate range and operating rooms may be allocated so that the deviation in utilization rate for each operating room is reduced.

Accordingly, during regular surgery hours (e.g., the same hours as the regular working hours of workers within a hospital, which may be 08:00 to 17:00, 08:30 to 17:30, etc.), operating rooms are allocated to increase a utilization rate of operating rooms with a utilization rate below a certain standard, and outside of regular surgery hours, operating rooms are allocated to lower a utilization rate of operating rooms with a utilization rate higher than a certain standard, allowing the hospital to operate operating rooms more efficiently and operate human and material resources more efficiently.

The embodiments described above may be implemented through various devices. For example, the embodiments may be implemented by hardware, firmware, software, or a combination thereof.

According to an embodiment, by calculating a utilization rate of each operating room by time period and using an average utilization rate of each operating room by time period for a period set by a user, an operating room utilization rate may be adjusted to an appropriate range and operating rooms may be allocated so that the deviation in utilization rate for each operating room is reduced.

Accordingly, during regular surgery hours, operating rooms are allocated to increase a utilization rate of operating rooms with a utilization rate below a certain standard, and outside of regular surgery hours, operating rooms are allocated to lower a utilization rate of operating rooms with a utilization rate higher than a certain standard, allowing the hospital to operate operating rooms more efficiently and operate human and material resources more efficiently.

In addition, various effects directly or indirectly identified through this document may be provided.

The embodiments described above may be implemented by hardware components, software components, and/or any combination thereof. For example, the devices, the methods, and components described in the embodiments may be implemented by using general-purpose computers or special-purpose computers, such as a processor, a controller, an arithmetic logic unit (ALU), a digital signal processor, a microcomputer, a field programmable gate array (FPGA), a programmable logic unit (PLU), a microprocessor, or any other devices which may execute and respond to instructions. A processing apparatus may execute an operating system (OS) and a software application executed in the OS. Also, the processing apparatus may access, store, operate, process, and generate data in response to the execution of software. For convenience of understanding, it may be described that one processing apparatus is used. However, one of ordinary skill in the art will understand that the processing apparatus may include a plurality of processing elements and/or various types of processing elements. For example, the processing apparatus may include a plurality of processors or a processor and a controller. Also, other processing configurations, such as a parallel processor, are also possible.

The software may include computer programs, code, instructions, or any combination thereof, and may construct the processing apparatus for desired operations or may independently or collectively command the processing apparatus. In order to be interpreted by the processing apparatus or to provide commands or data to the processing apparatus, the software and/or data may be permanently or temporarily embodied in any types of machines, components, physical devices, virtual equipment, computer storage mediums, or transmitted signal waves. The software may be distributed over network coupled computer systems so that it may be stored and executed in a distributed fashion. The software and/or data may be recorded in a computer-readable recording medium.

A method according to an embodiment may be implemented as program instructions that can be executed by various computer devices, and recorded on a computer-readable recording medium. The computer-readable recording medium may include program instructions, data files, data structures or a combination thereof. Program instructions recorded on the medium may be particularly designed and structured for embodiments or available to one of ordinary skill in a field of computer software. Examples of the computer-readable recording medium include magnetic media, such as a hard disc, a floppy disc, and magnetic tape; optical media, such as a compact disc-read only memory (CD-ROM) and a digital versatile disc (DVD); magneto-optical media, such as floptical discs; and hardware devices specially configured to store and execute program instructions, such as ROM, random-access memory (RAM), a flash memory, etc. Program instructions may include, for example, high-level language code that can be executed by a computer using an interpreter, as well as machine language code made by a complier.

In concluding the detailed description, those skilled in the art will appreciate that many variations and modifications may be made to the preferred embodiments within the scope of the appended claims.

**Claims**

1. An operating room allocation apparatus comprising:

> an information obtaining unit configured to obtain information about patient's entry and exit times in each operating room;
> a first calculation unit configured to calculate a utilization rate of the operating room for each certain time period based on the patient's entry and exit times,
> wherein the first calculation unit divides a time from time information specified as the entry time to time information specified as the exit time by preset time intervals, calculate a ratio of the operating room used for each of the preset time intervals, and calculate a utilization rate of the operating room; and
a second calculation unit configured to receive certain time information from a user and calculate an average utilization rate by time period in the received time information,
wherein, when the entry time is x1:y1, the exit time is x2:y2, x1 = x2, and the certain time is set at 30-minute intervals,
the first calculation unit calculates a utilization rate from x1:00 to x1:30 as $\frac{(y2 - y1)}{30}$ when minute information y1 of the entry time and minute information y2 of the exit time are 30 minutes before or 30 minutes,
calculates a utilization rate from x1:30 to x1:60 as $\frac{(y2 - y1)}{30}$ when minute information y1 of the entry time and minute information y2 of the exit time are 30 minutes after, and
calculates a utilization rate from x1:00 to x1:30 as $\frac{(30 - y1)}{30}$ and calculates a utilization rate from x1:30 to x1:60 as $\frac{(y2-30)}{30}$ when minute information y1 of the entry time is 30 minutes before or 30 minutes, and minute information y2 of the exit time is 30 minutes after.

2. The operating room allocation apparatus of claim 1, wherein the information obtaining unit further obtains information about downtime of the operating room, and
the first calculation unit specify a time obtained by adding the downtime to the exit time as a final exit time, calculate a time from the entry time to the final exit time as a time the operating room was used, and calculate a utilization rate of the operating room for the each certain time period.

3. The operating room allocation apparatus according to claim 1 or 2, wherein, when the entry time is x1:y1, the exit time is x2:y2, x1 ≠ x2, and the certain time is set at 30-minute intervals,

the first calculation unit calculates a utilization rate from x1:00 to x1:30 as $\frac{(30 - y1)}{30}$ and calculates a utilization rate from x1:30 to x1:60 as 1 when minute information y1 of the entry time is 30 minutes before or 30 minutes,
calculates a utilization rate from x1:30 to x1:60 as $\frac{(60- y1)}{30}$ when minute information y1 of the entry time is 30 minutes after,
calculates a utilization rate from x2:00 to x2:30 as $\frac{y2}{30}$ when minute information y2 of the exit time is 30 minutes before or 30 minutes,
calculates a utilization rate from x2:00 to x2:30 as 1 and calculates a utilization rate from x2:30 to x2:60 as $\frac{(y2-30)}{30}$ when minute information y2 of the exit time is 30 minutes after, and
calculates a utilization rate from a:00 to a:30 as 1, and a utilization rate from a:30 to a:60 as 1 for an integer 'a' that satisfies condition x1< a <x2.

4. The operating room allocation apparatus according to any of claims 1-3, wherein, when x1 > x2,

the first calculation unit calculates a utilization rate from b:00 to b:30 as 1, and a utilization rate from b:30 to b:60 as 1 for an integer b that satisfies condition x1< b <23, and
calculates a utilization rate from c:00 to c:30 as 1, and a utilization rate from c:30 to c:60 as 1 for an integer c that satisfies condition 0< c <x2.

5. The operating room allocation apparatus according to any of claims 1-4, wherein, when the entry time is x1:y1, the exit time is x2:y2, x1 = x2, and the certain time is set at 1 hour intervals,
the first calculation unit calculates a utilization rate at x1 o'clock as $\frac{(y2 - y1)}{60}$ .

6. The operating room allocation apparatus according to any of claims 1-5, wherein, when the entry time is x1:y1, the exit time is x2:y2, x1 ≠ x2, and the certain time is set at 1 hour intervals,

the first calculation unit calculates a utilization rate at x1 o'clock as $\frac{(60-y )}{60}$ and calculates a utilization rate at x2 o'clock as $\frac{y2}{60}$ , and
calculates a utilization rate at 'a' o'clock as 1 for an integer 'a' that satisfies condition x1< a <x2.

7. The operating room allocation apparatus according to any of claims 1-6, wherein, when x1 > x2,

the first calculation unit calculates a utilization rate at b o'clock as 1 for an integer b that satisfies condition x1< b <23, and
calculates a utilization rate at c o'clock as 1 for an integer c that satisfies condition 0< c <x2.

8. The operating room allocation apparatus according to any of claims 1-7, wherein the second calculation unit receives time information of at least one of day of the week, time period, and period from a user, filters a utilization rate by time period that satisfies the received time information, and then calculates an average utilization rate by time period by averaging the filtered utilization rate by time period in the received time information.

9. The operating room allocation apparatus according to any of claims 1-8, further comprising:

an operating room allocation unit configured to allocate, from among operating rooms, an operating room where a patient can be operated on based on an average utilization rate of each of the operating rooms by time period,
wherein the operating room allocation unit preferably allocates an operating room where a patient can be operated on from among the operating rooms, so that an average utilization rate of each of the operating rooms by time period falls within a certain range.

10. An operating room allocation method performed by an operating room allocation apparatus, the operating room allocation method comprising:

obtaining information about patient's entry and exit times in an operating room;
calculating a utilization rate of the operating room for each certain time period based on the patient's entry and exit times, wherein calculating the utilization rate comprises dividing a time from time information specified as the entry time to time information specified as the exit time by preset time intervals, and calculating a ratio of the operating room used for each of the preset time intervals; and
receiving certain time information from a user and calculating an average utilization rate by time period in the received time information, wherein, when the entry time is x1:y1, the exit time is x2:y2, x1 = x2, and the certain time is set at 30-minute intervals,
a utilization rate from x1:00 to x1:30 is calculated

as $\frac{(y2 - y1)}{30}$ when minute information y1 of the entry time and minute information y2 of the exit time are 30 minutes before or 30 minutes, a utilization rate from x1:30 to x1:60 is calculated

as $\frac{(y2 - y1)}{30}$ when minute information y1 of the entry time and minute information y2 of the exit time are 30 minutes after, and
a utilization rate from x1:00 to x1:30 is calculated

as $\frac{(30 - y1)}{30}$ and a utilization rate from x1:30 to x1:60 is calculated as $\frac{(y2 - 30)}{30}$ when minute

information y1 of the entry time is 30 minutes before or 30 minutes, and minute information y2 of the exit time is 30 minutes after.

11. The operating room allocation method of claim 10, wherein the obtaining comprises:

obtaining information about downtime of the operating room, and
the calculating of the utilization rate of the operating room comprises:
calculating the utilization rate of the operating room for the each certain time period by additionally considering the downtime, and/or
wherein the calculating of the average utilization rate by time period comprises:
receiving time information of at least one of day of the week, time period, and period from a user, filtering a utilization rate by time period that satisfies the received time information, and then calculating an average utilization rate by time period by averaging the filtered utilization rate by time period in the received time information.

12. The operating room allocation method of any of claim 10 or 11, further comprising:
allocating, from among operating rooms, an operating room where a patient can be operated on based on the average utilization rate, wherein the allocating preferably comprises:
allocating an operating room where a patient can be operated on from among the operating rooms, so that an average utilization rate of each of the operating rooms by time period falls within a certain range.

13. A non-transitory computer-readable recording medium having recorded thereon a computer program, the non-transitory computer-readable recording medium having recorded thereon instructions which, when executed by a processor, cause the processor to:

obtain information about patient's entry and exit times in an operating room;
calculate a utilization rate of the operating room for each certain time period based on the patient's entry and exit times, wherein calculating the utilization rate comprises dividing a time from time information specified as the entry time to time information specified as the exit time by preset time intervals, and calculating a ratio of the operating room used for each of the preset time intervals,

wherein, when the entry time is x1:y1, the exit time is x2:y2, x1 = x2, and the certain time is set at 30-minute intervals,

a utilization rate from x1:00 to x1:30 is calculated as $\frac{(y2 - y1)}{30}$ when minute information y1 of the entry time and minute information y2 of the exit time are 30 minutes before or 30 minutes,

a utilization rate from x1:30 to x1:60 is calculated as $\frac{(y2 - y1)}{30}$ when minute information y1 of the entry time and minute information y2 of the exit time are 30 minutes after, and

a utilization rate from x1:00 to x1:30 is calculated as $\frac{(30 - y1)}{30}$ and a utilization rate from x1:30 to x1:60 is calculated as $\frac{(y2 - 30)}{30}$ when minute information y1 of the entry time is 30 minutes before or 30 minutes, and minute information y2 of the exit time is 30 minutes after; and

receive certain time information from a user and calculate an average utilization rate by time period in the received time information.

**Patentansprüche**

1. Vorrichtung zur Zuweisung von Operationssälen, umfassend:

eine Informationserfassungseinheit, die dazu konfiguriert ist, Informationen über Ein- und Austrittszeiten von Patienten in jedem Operationssaal zu erfassen;

eine erste Berechnungseinheit, die so konfiguriert ist, dass sie eine Nutzungsrate des Operationssaals für jeden bestimmten Zeitraum auf der Grundlage der Ein- und Austrittszeiten des Patienten berechnet,

wobei die erste Berechnungseinheit eine Zeit von der als Eintrittszeit angegebenen Zeitinformation bis zur als Austrittszeit angegebenen Zeitinformation in voreingestellte Zeitintervalle unterteilt, ein Verhältnis des genutzten Operationssaals für jedes der voreingestellten Zeitintervalle berechnet und eine Nutzungsrate des Operationssaals berechnet; und

eine zweite Berechnungseinheit, die so konfiguriert ist, dass sie bestimmte Zeitinformationen von einem Nutzer empfängt und eine durchschnittliche Nutzungsrate nach Zeitabschnitt in den empfangenen Zeitinformationen berechnet,

wobei, wenn die Eintrittszeit x1:y1 ist, die Austrittszeit x2:y2 ist, x1 = x2 ist und die bestimmte Zeit in Intervallen von 30 Minuten festgelegt ist,

die erste Berechnungseinheit eine Nutzungsrate von x1:00 bis x1:30 als $\frac{(y2 - y1)}{30}$ berechnet, wenn die Minutenangabe y1 der Eintrittszeit und die Minutenangabe y2 der Austrittszeit bei 30 Minuten davor oder bei 30 Minuten liegen,

eine Nutzungsrate von x1:30 bis x1:60 als $\frac{(y2 - y1)}{30}$ berechnet, wenn die Minutenangabe y1 der Eintrittszeit und die Minutenangabe y2 der Austrittszeit bei 30 Minuten danach liegen,

und

eine Nutzungsrate von x1:00 bis x1:30 als $\frac{(30 - y1)}{30}$ berechnet und eine Nutzungsrate von x1:30 bis x1:60 als $\frac{(y2 - 30)}{30}$ berechnet, wenn die Minutenangabe y1 der Eintrittszeit bei 30 Minuten davor oder bei 30 Minuten liegt und die Minutenangabe y2 der Austrittszeit bei 30 Minuten danach liegt.

2. Operationssaal-Zuweisungsvorrichtung nach Anspruch 1, wobei die Informationserfassungseinheit ferner Informationen über die Ausfallzeit des Operationssaals erfasst und

die erste Berechnungseinheit eine Zeit, die durch Addition der Ausfallzeit zur Austrittszeit erhalten wird, als endgültige Austrittszeit festlegt, eine Zeit von der Eintrittszeit bis zur endgültigen Austrittszeit als Zeit berechnet, in der der Operationssaal genutzt wurde, und eine Nutzungsrate des Operationssaals für jeden bestimmten Zeitabschnitt berechnet.

3. Operationssaal-Zuweisungsvorrichtung nach Anspruch 1 oder 2, wobei, wenn die Eintrittszeit x1:y1 ist, die Austrittszeit x2:y2 ist, x1 ≠ x2 ist und die bestimmte Zeit in Intervallen von 30 Minuten festgelegt ist,

die erste Berechnungseinheit eine Nutzungsrate von x1:00 bis x1:30 als $\frac{(30 - y1)}{30}$ berechnet und eine Nutzungsrate von x1:30 bis x1:60 als 1 berechnet, wenn die Minutenangabe y1 der Eintrittszeit bei 30 Minuten davor oder bei 30 Minuten liegt,

eine Nutzungsrate von x1:30 bis x1:60 als $\frac{(60 - y1)}{30}$ berechnet, wenn die Minutenangabe y1 der Eintrittszeit bei 30 Minuten danach liegt,

eine Nutzungsrate von x2:00 bis x2:30 als $\dfrac{y2}{30}$ berechnet, wenn die Minutenangabe y2 der Austrittszeit bei 30 Minuten davor oder bei 30 Minuten liegt,

eine Nutzungsrate von x2:00 bis x2:30 als 1 berechnet und eine Nutzungsrate von x2:30 bis x2:60 als $\dfrac{(y2-30)}{30}$ berechnet, wenn die Minutenangabe y2 der Austrittszeit bei 30 Minuten danach liegt, und

eine Nutzungsrate von a:00 bis a:30 als 1 berechnet und eine Nutzungsrate von a:30 bis a:60 als 1 berechnet, für eine ganze Zahl "a", die die Bedingung x1< a <x2 erfüllt.

4. Operationssaal-Zuweisungsvorrichtung nach einem der Ansprüche 1-3, wobei, wenn x1 > x2,

die erste Berechnungseinheit eine Nutzungsrate von b:00 bis b:30 als 1 und eine Nutzungsrate von b:30 bis b:60 als 1 für eine ganze Zahl b berechnet, die die Bedingung x1 < b < 23 erfüllt, und

eine Nutzungsrate von c:00 bis c:30 als 1 und eine Nutzungsrate von c:30 bis c:60 als 1 für eine ganze Zahl c berechnet, die die Bedingung 0 < c < x2 erfüllt.

5. Operationssaal-Zuweisungsvorrichtung nach einem der Ansprüche 1-4, wobei, wenn die Eintrittszeit x1:y1 ist, die Austrittszeit x2:y2 ist, x1 = x2 ist und die bestimmte Zeit in Intervallen von 1 Stunde festgelegt ist,

die erste Berechnungseinheit eine Nutzungsrate um

x1 Uhr als $\dfrac{(y2-y1)}{60}$ berechnet.

6. Operationssaal-Zuweisungsvorrichtung nach einem der Ansprüche 1-5, wobei, wenn die Eintrittszeit x1:y1 ist, die Austrittszeit x2:y2 ist, x1 ≠ x2 ist und die bestimmte Zeit in Intervallen von 1 Stunde festgelegt ist,

die erste Berechnungseinheit eine Nutzungsrate um x1 Uhr als $\dfrac{(60-y\ )}{60}$ und eine Nutzungsrate um x2 Uhr als $\dfrac{y2}{60}$ berechnet und

eine Nutzungsrate um "a" Uhr als 1 für eine ganze Zahl "a" berechnet, die die Bedingung x1< a <x2 erfüllt.

7. Operationssaal-Zuweisungsvorrichtung nach einem der Ansprüche 1- 6, wobei, wenn x1 > x2,

die erste Berechnungseinheit eine Nutzungsrate um b Uhr als 1 für eine ganze Zahl b berechnet, die die Bedingung x1< b <23 erfüllt, und eine Nutzungsrate um c Uhr als 1 für eine ganze Zahl c berechnet, die die Bedingung 0< c <x2 erfüllt.

8. Operationssaal-Zuweisungsvorrichtung nach einem der Ansprüche 1- 7, wobei die zweite Berechnungseinheit Zeitinformationen zu mindestens einem der folgenden Punkte von einem Nutzer empfängt: Wochentag, Zeitabschnitt und Abschnitt, eine Nutzungsrate nach Zeitabschnitt filtert, der die empfangenen Zeitinformationen erfüllt, und dann eine durchschnittliche Nutzungsrate nach Zeitabschnitt berechnet, indem sie die gefilterte Nutzungsrate nach Zeitabschnitt in den empfangenen Zeitinformationen mittelt.

9. Operationssaal-Zuweisungsvorrichtung nach einem der Ansprüche 1-8, die ferner umfasst:

eine Operationssaal-Zuweisungseinheit, die so konfiguriert ist, dass sie aus den Operationssälen einen Operationssaal zuweist, in dem ein Patient operiert werden kann, basierend auf einer durchschnittlichen Nutzungsrate jedes der Operationssäle nach Zeitabschnitt,

wobei die Operationssaal-Zuweisungseinheit vorzugsweise einen Operationssaal, in dem ein Patient operiert werden kann, aus den Operationssälen so zuweist, dass eine durchschnittliche Nutzungsrate jedes Operationssaals pro Zeitabschnitt in einen bestimmten Bereich fällt.

10. Verfahren zur Zuweisung von Operationssälen, das von einer Operationssaal-Zuweisungsvorrichtung durchgeführt wird, wobei das Verfahren zur Zuweisung von Operationssälen umfasst:

Erfassen von Informationen über Ein- und Austrittszeiten von Patienten in einem Operationssaal;

Berechnen einer Nutzungsrate des Operationssaals für jeden bestimmten Zeitabschnitt auf der Grundlage der Ein- und Austrittszeiten des Patienten, wobei die Berechnung der Nutzungsrate das Unterteilen einer Zeit von der als Eintrittszeit angegebenen Zeitinformation bis zur als Austrittszeit angegebenen Zeitinformation in voreingestellte Zeitintervalle umfasst, und Berechnen eines Verhältnisses des genutzten Operationssaals für jedes der voreingestellten Zeitintervalle; und

Empfangen bestimmter Zeitinformationen von einem Nutzer und Berechnen einer durchschnittlichen Nutzungsrate nach Zeitabschnitt in den empfangenen Zeitinformationen,

wobei, wenn die Eintrittszeit x1:y1 ist, die Austrittszeit x2:y2 ist, x1 = x2 ist und die bestimmte Zeit in Intervallen von 30 Minuten festgelegt ist, eine Nutzungsrate von x1:00 bis x1:30 als

$$\frac{(y2 - y1)}{30}$$ berechnet wird, wenn die Minutenangabe y1 der Eintrittszeit und die Minutenangabe y2 der Austrittszeit bei 30 Minuten davor oder bei 30 Minuten liegen,
eine Nutzungsrate von x1:30 bis x1:60 als

$$\frac{(y2 - y1)}{30}$$ berechnet wird, wenn die Minutenangabe y1 der Eintrittszeit und die Minutenangabe y2 der Austrittszeit bei 30 Minuten danach liegen,
und
eine Nutzungsrate von x1:00 bis x1:30 als

$$\frac{(30 - y1)}{30}$$ berechnet wird und eine Nutzungsrate von x1:30 bis x1:60 als $\frac{(y2-30)}{30}$ berechnet wird, wenn die Minutenangabe y1 der Eintrittszeit bei 30 Minuten davor oder bei 30 Minuten liegt und die Minutenangabe y2 der Austrittszeit bei 30 Minuten danach liegt.

11. Verfahren zur Zuweisung von Operationssälen nach Anspruch 10, wobei das Erfassen umfasst:

Erfassen von Informationen über Ausfallzeiten des Operationssaals, und
das Berechnen der Nutzungsrate des Operationssaals umfasst:
Berechnen der Nutzungsrate des Operationssaals für jeden bestimmten Zeitabschnitt unter zusätzlicher Berücksichtigung der Ausfallzeit, und/oder wobei das Berechnen der durchschnittlichen Nutzungsrate pro Zeitabschnitt umfasst:
Empfangen von Zeitinformationen zu mindestens einem der folgenden Punkte von einem Nutzer: Wochentag, Zeitabschnitt und Abschnitt, Filtern einer Nutzungsrate nach Zeitabschnitt, der den empfangenen Zeitinformationen entspricht, und anschließendes Berechnen einer durchschnittlichen Nutzungsrate nach Zeitabschnitt durch Mitteln der gefilterten Nutzungsrate nach Zeitabschnitt in den empfangenen Zeitinformationen.

12. Verfahren zur Zuweisung von Operationssälen nach Anspruch 10 oder 11, das ferner umfasst:
Zuweisen eines Operationssaals, in dem ein Patient operiert werden kann, aus den Operationssälen auf der Grundlage der durchschnittlichen Nutzungsrate, wobei das Zuweisen vorzugsweise umfasst:
Zuweisen eines Operationssaals, in dem ein Patient operiert werden kann, aus den Operationssälen, so dass die durchschnittliche Nutzung jedes Operationssaals pro Zeitabschnitt innerhalb eines bestimmten Bereichs liegt.

13. Nicht-transitorisches, computerlesbares Aufzeichnungsmedium, auf dem ein Computerprogramm aufgezeichnet ist, wobei auf dem nicht-transitorischen, computerlesbaren Aufzeichnungsmedium Befehle aufgezeichnet sind, die, wenn sie von einem Prozessor ausgeführt werden, den Prozessor dazu veranlassen:

Informationen über die Ein- und Austrittszeiten von Patienten in einem Operationssaal zu erfassen;
eine Nutzungsrate des Operationssaals für jeden bestimmten Zeitabschnitt auf der Grundlage der Ein- und Austrittszeiten des Patienten zu berechnen, wobei die Berechnung der Nutzungsrate das Unterteilen einer Zeit von einer als Eintrittszeit angegebenen Zeitinformation bis zu einer als Austrittszeit angegebene Zeitinformation in voreingestellte Zeitintervalle und das Berechnen eines Verhältnisses des für jedes der voreingestellten Zeitintervalle genutzten Operationssaals umfasst,
wobei, wenn die Eintrittszeit x1:y1 ist, die Austrittszeit x2:y2 ist, x1 = x2 ist und der bestimmte Zeitraum auf 30-Minuten-Intervalle festgelegt ist,
eine Nutzungsrate von x1:00 bis x1:30 als

$$\frac{(y2 - y1)}{30}$$ berechnet wird, wenn die Minutenangabe y1 der Eintrittszeit und die Minutenangabe y2 der Austrittszeit bei 30 Minuten davor oder bei 30 Minuten liegen,
eine Nutzungsrate von x1:30 bis x1:60 als

$$\frac{(y2 - y1)}{30}$$ berechnet wird, wenn die Minutenangabe y1 der Eintrittszeit und die Minutenangabe y2 der Austrittszeit bei 30 Minuten danach liegen,
und
eine Nutzungsrate von x1:00 bis x1:30 als

$$\frac{(30 - y1)}{30}$$ berechnet wird und eine Nutzungsrate von x1:30 bis x1:60 als $\frac{(y2-30)}{30}$ berechnet wird, wenn die Minutenangabe y1 der Eintrittszeit bei 30 Minuten davor oder bei 30 Minuten liegt und die Minutenangabe y2 der Austrittszeit bei 30 Minuten danach liegt; und

bestimmte Zeitinformationen von einem Nutzer zu empfangen und eine durchschnittliche Nutzungsrate nach Zeitabschnitt in den empfangenen Zeitinformationen zu berechnen.

**Revendications**

1. Appareil d'attribution de salle d'opération, comprenant :

   une unité d'obtention d'informations configurée pour obtenir des informations concernant des heures d'entrée et de sortie d'un patient dans chaque salle d'opération ;
   une première unité de calcul configurée pour calculer un taux d'utilisation de la salle d'opération pour chaque certaine période de temps sur la base des heures d'entrée et de sortie du patient,
   dans lequel la première unité de calcul divise un temps allant des informations temporelles spécifiées en tant que l'heure d'entrée à des informations temporelles spécifiées en tant que l'heure de sortie par des intervalles de temps prédéfinis, calcule un ratio de la salle d'opération utilisée pour chacun des intervalles de temps prédéfinis, et calcule un taux d'utilisation de la salle d'opération ; et
   une seconde unité de calcul configurée pour recevoir certaines informations temporelles en provenance d'un utilisateur et pour calculer un taux d'utilisation moyen par période de temps dans les informations temporelles reçues,
   dans lequel, lorsque l'heure d'entrée est x1:y1, que l'heure de sortie est x2:y2, que x1 = x2, et que le certain temps est fixé à des intervalles de 30 minutes,
   la première unité de calcul calcule un taux d'utilisation allant de x1:00 à x1:30 comme étant

   $\dfrac{(y2-y1)}{30}$ lorsque des informations de minutes y1 de l'heure d'entrée et des informations de minutes y2 de l'heure de sortie sont 30 minutes avant ou 30 minutes,

   calcule un taux d'utilisation allant de x1:30 à x1:60 comme étant $\dfrac{(y2-y1)}{30}$ lorsque des informations de minutes y1 de l'heure d'entrée et des informations de minutes y2 de l'heure de sortie sont 30 minutes après, et

   calcule un taux d'utilisation allant de x1:00 à x1:30 comme étant $\dfrac{(30-y1)}{30}$ et calcule un taux d'utilisation allant de x1:30 à x1:60 comme

   étant $\dfrac{(y2-30)}{30}$ lorsque des informations de minutes y1 de l'heure d'entrée sont 30 minutes avant ou 30 minutes, et que des informations de minutes y2 de l'heure de sortie sont 30 minutes après.

2. Appareil d'attribution de salle d'opération selon la revendication 1, dans lequel l'unité d'obtention d'informations obtient en outre des informations concernant un temps d'inactivité de la salle d'opération, et la première unité de calcul spécifie un temps obtenu en ajoutant le temps d'inactivité à l'heure de sortie en tant qu'heure de sortie finale, calcule un temps allant de l'heure d'entrée à l'heure de sortie finale en tant que temps d'utilisation de la salle d'opération, et calcule un taux d'utilisation de la salle d'opération pour chaque certaine période de temps.

3. Appareil d'attribution de salle d'opération selon la revendication 1 ou 2, dans lequel, lorsque l'heure d'entrée est x1:y1, que l'heure de sortie est x2:y2, que x1 ≠ x2, et que le certain temps est fixé à des intervalles de 30 minutes,

   la première unité de calcul calcule un taux d'utilisation allant de x1:00 à x1:30 comme étant

   $\dfrac{(30-y1)}{30}$ et calcule un taux d'utilisation allant de x1:30 à x1:60 comme étant 1 lorsque des informations de minutes y1 de l'heure d'entrée sont 30 minutes avant ou 30 minutes,

   calcule un taux d'utilisation allant de x1:30 à x1:60 comme étant $\dfrac{(60-y1)}{30}$ lorsque des informations de minutes y1 de l'heure d'entrée sont 30 minutes après,

   calcule un taux d'utilisation allant de x2:00 à x2:30 comme étant $\dfrac{y2}{30}$ lorsque des informations de minutes y2 de l'heure de sortie sont 30 minutes avant ou 30 minutes,

   calcule un taux d'utilisation allant de x2:00 à x2:30 comme étant 1 et calcule un taux d'utilisation allant de x2:30 à x2:60 comme étant

   $\dfrac{(y2-30)}{30}$ lorsque des informations de minutes y2 de l'heure de sortie sont 30 minutes après, et calcule un taux d'utilisation allant de a:00 à a:30 comme étant 1, et un taux d'utilisation allant de a:30 à a:60 comme étant 1 pour un nombre entier 'a' qui satisfait la condition x1 < a < x2.

4. Appareil d'attribution de salle d'opération selon l'une quelconque des revendications 1 à 3, dans lequel, lorsque x1 > x2,

la première unité de calcul calcule un taux d'utilisation allant de b:00 à b:30 comme étant 1, et un taux d'utilisation allant de b:30 à b:60 comme étant 1 pour un nombre entier b qui satisfait la condition x1 < b < 23, et calcule un taux d'utilisation allant de c:00 à c:30 comme étant 1, et un taux d'utilisation allant de c:30 à c:60 comme étant 1 pour un nombre entier c qui satisfait la condition 0 < c < x2.

5. Appareil d'attribution de salle d'opération selon l'une quelconque des revendications 1 à 4, dans lequel, lorsque l'heure d'entrée est x1:y1, que l'heure de sortie est x2:y2, que x1 = x2, et que le certain temps est fixé à des intervalles de 1 heure, la première unité de calcul calcule un taux d'utilisation à x1 heure comme étant $\frac{(y2-y1)}{60}$ .

6. Appareil d'attribution de salle d'opération selon l'une quelconque des revendications 1 à 5, dans lequel, lorsque l'heure d'entrée est x1:y1, que l'heure de sortie est x2:y2, que x1 ≠ x2, et que le certain temps est fixé à des intervalles de 1 heure,

la première unité de calcul calcule un taux d'utilisation à x1 heure comme étant $\frac{(60-y)}{60}$ et calcule un taux d'utilisation à x2 heure comme étant $\frac{y2}{60}$ , et calcule un taux d'utilisation à 'a' heure comme étant 1 pour un nombre entier 'a' qui satisfait la condition x1 < a < x2.

7. Appareil d'attribution de salle d'opération selon l'une quelconque des revendications 1 à 6, dans lequel, lorsque x1 > x2,

la première unité de calcul calcule un taux d'utilisation à b heure comme étant 1 pour un nombre entier b qui satisfait une condition x1 < b < 23, et calcule un taux d'utilisation à c heure comme étant 1 pour un nombre entier c qui satisfait la condition 0 < c < x2.

8. Appareil d'attribution de salle d'opération selon l'une quelconque des revendications 1 à 7, dans lequel la seconde unité de calcul reçoit des informations temporelles d'au moins l'un d'un jour de la semaine, d'une période de temps, et d'une période en provenance d'un utilisateur, filtre un taux d'utilisation par période de temps qui satisfait les informations temporelles reçues, et calcule ensuite un taux d'utilisation moyen par période de temps en faisant la moyenne du taux d'utilisation filtré par période de temps des informations temporelles reçues.

9. Appareil d'attribution de salle d'opération selon l'une quelconque des revendications 1 à 8, comprenant en outre :

une unité d'attribution de salle d'opération configurée pour attribuer, parmi des salles d'opération, une salle d'opération dans laquelle un patient peut être opéré sur la base d'un taux d'utilisation moyen de chacune des salles d'opération par période de temps, dans lequel l'unité d'attribution de salle d'opération attribue de préférence une salle d'opération dans laquelle un patient peut être opéré parmi les salles d'opération, de sorte qu'un taux d'utilisation moyen de chacune des salles d'opérations par période de temps s'inscrive dans une certaine plage.

10. Procédé d'attribution de salle d'opération mis en œuvre par un appareil d'attribution de salle d'opération, le procédé d'attribution de salle d'opération comprenant les étapes consistant à :

obtenir des informations concernant des heures d'entrée et de sortie d'un patient dans une salle d'opération ; calculer un taux d'utilisation de la salle d'opération pour chaque certaine période de temps sur la base des heures d'entrée et de sortie du patient, dans lequel l'étape de calcul du taux d'utilisation comprend de diviser un temps allant des informations temporelles spécifiées en tant que l'heure d'entrée à des informations temporelles spécifiées en tant que l'heure de sortie par des intervalles de temps prédéfinis, et à calculer un ratio de la salle d'opération utilisée pour chacun des intervalles de temps prédéfinis ; et recevoir certaines informations temporelles en provenance d'un utilisateur et calculer un taux d'utilisation moyen par période de temps des informations temporelles reçues, dans lequel, lorsque l'heure d'entrée est x1:y1, que l'heure de sortie est x2:y2, que x1 = x2, et que le certain temps est fixé à des intervalles de 30 minutes, un taux d'utilisation allant de x1:00 à x1:30 est calculé comme étant $\frac{(y2-y1)}{30}$ lorsque des informations de minutes y1 de l'heure d'entrée et des informations de minutes y2 de l'heure de sortie sont 30 minutes avant ou 30 minutes, un taux d'utilisation allant de x1:30 à x1:60 est calculé comme étant $\frac{(y2-y1)}{30}$ lorsque des informations de minutes y1 de l'heure d'entrée et des informations de minutes y2 de l'heure de sortie sont 30 minutes après, et

un taux d'utilisation allant de x1:00 à x1:30 est calculé comme étant $\frac{(30-y1)}{30}$ et un taux d'utilisation allant de x1:30 à x1:60 est calculé comme étant $\frac{(y2-30)}{30}$ lorsque des informations de minutes y1 de l'heure d'entrée sont 30 minutes avant ou 30 minutes, et que des informations de minutes y2 de l'heure de sortie sont 30 minutes après.

11. Procédé d'attribution de salle d'opération selon la revendication 10, dans lequel l'étape d'obtention comprend :

obtenir des informations concernant un temps d'inactivité de la salle d'opération, et l'étape de calcul du taux d'utilisation de la salle d'opération comprend :
calculer le taux d'utilisation de la salle d'opération pour chaque certaine période de temps par considération en plus le temps d'inactivité, et/ou dans lequel l'étape de calcul du taux d'utilisation moyen par période de temps comprend :
recevoir des informations temporelles d'au moins l'un d'un jour de la semaine, d'une période de temps, et d'une période en provenance d'un utilisateur, filtrer un taux d'utilisation par période de temps qui satisfait les informations temporelles reçues, et calculer ensuite un taux d'utilisation moyen par période de temps en faisant la moyenne du taux d'utilisation filtré par période de temps des informations temporelles reçues.

12. Procédé d'attribution de salle d'opération selon l'une quelconque des revendications 10 et 11, comprenant en outre les étapes consistant à :
attribuer, parmi des salles d'opération, une salle d'opération dans laquelle un patient peut être opéré sur la base du taux d'utilisation moyen, dans lequel l'attribution comprend de préférence :
attribuer une salle d'opération dans laquelle un patient peut être opéré parmi les salles d'opération, de sorte qu'un taux d'utilisation moyen de chacune des salles d'opération par période de temps s'inscrive dans une certaine plage.

13. Support d'enregistrement non transitoire lisible par ordinateur comportant un programme d'ordinateur enregistré dessus, le support d'enregistrement non transitoire lisible par ordinateur comportant des instructions enregistrées dessus qui, lorsqu'elles sont exécutées par un processeur, amènent le processeur à :

obtenir des informations concernant des heures d'entrée et de sortie d'un patient dans une salle d'opération ;
calculer un taux d'utilisation de la salle d'opération pour chaque certaine période de temps sur la base des heures d'entrée et de sortie du patient, dans lequel l'étape de calcul du taux d'utilisation comprend de diviser un temps allant des informations temporelles spécifiées en tant que l'heure d'entrée à des informations temporelles spécifiées en tant que l'heure de sortie par des intervalles de temps prédéfinis, et de calculer un ratio de la salle d'opération utilisée pour chacun des intervalles de temps prédéfinis ;
dans lequel, lorsque l'heure d'entrée est x1:y1, que l'heure de sortie est x2:y2, que x1 = x2, et le certain temps est fixé à des intervalles de 30 minutes,

un taux d'utilisation allant de x1:00 à x1:30 est calculé comme étant $\frac{(y2-y1)}{30}$ lorsque des informations de minutes y1 de l'heure d'entrée et des informations de minutes y2 de l'heure de sortie sont 30 minutes avant ou 30 minutes,

un taux d'utilisation allant de x1:30 à x1:60 est calculé comme étant $\frac{(y2-y1)}{30}$ lorsque des informations de minutes y1 de l'heure d'entrée et des informations de minutes y2 de l'heure de sortie sont 30 minutes après, et

un taux d'utilisation allant de x1:00 à x1:30 est calculé comme étant $\frac{(30-y1)}{30}$ et un taux d'utilisation allant de x1:30 à x1:60 est calculé comme étant $\frac{(y2-30)}{30}$ lorsque des informations de minutes y1 de l'heure d'entrée sont 30 minutes avant ou 30 minutes, et que des informations de minutes y2 de l'heure de sortie sont 30 minutes après ; et

recevoir certaines informations temporelles en provenance d'un utilisateur et calculer un taux d'utilisation moyen par période de temps des informations temporelles reçues.

FIG. 1

100

110

INFORMATION
OBTAINING UNIT

120

FIRST
CALCULATION UNIT

130

SECOND
CALCULATION UNIT

140

OPERATING ROOM
ALLOCATION UNIT

# FIG. 2

SELECT DAY OF WEEK | MON. | TUE. | WED. | THUR. | FRI.

SELECT PERIOD | THIS WEEK | ~1MONTH | ~3 MONTHS | ~12 MONTHS

SELECT TIME | 7am~10pm | 24H

| | 07:00 | 07:30 | 08:00 | 08:30 | 09:00 | 09:30 | 10:00 | 10:30 | 11:00 | 11:30 | 12:00 | 12:30 | 13:00 | 13:30 | 14:00 | 14:30 | 15:00 | 15:30 | 16:00 | 16:30 | 17:00 | 17:30 | 18:00 | 18:30 | 19:00 | 19:30 | 20:00 | 20:30 | 21:00 | 21:30 |
|---|---|---|---|---|---|---|---|---|---|---|---|---|---|---|---|---|---|---|---|---|---|---|---|---|---|---|---|---|---|---|
| E01 | - | - | 48 | 67 | 67 | 97 | 100 | 100 | 100 | 100 | 100 | 100 | 100 | 100 | 100 | 100 | 100 | 100 | 100 | 100 | 100 | 81 | 100 | 100 | 67 | 33 | 22 | - | - | - |
| E02 | - | - | 60 | 100 | 100 | 100 | 100 | 94 | 78 | 63 | 99 | 100 | 100 | 100 | 100 | 100 | 100 | 100 | 100 | 100 | 100 | 100 | 63 | 33 | 33 | 33 | 33 | 33 | 33 | 33 |
| E03 | - | - | 80 | 100 | 100 | 100 | 100 | 100 | 97 | 100 | 100 | 99 | 67 | 67 | 84 | 100 | 100 | 100 | 100 | 100 | 100 | 100 | 100 | 100 | 100 | 100 | 97 | 67 | 67 | 67 |
| E04 | - | - | 46 | 67 | 84 | 92 | 84 | 94 | 86 | 99 | 97 | 98 | 100 | 94 | 87 | 92 | 100 | 99 | 98 | 100 | 96 | 39 | 33 | 33 | 19 | - | - | - | - | - |
| E05 | - | - | 72 | 100 | 100 | 80 | 100 | 94 | 99 | 99 | 97 | 98 | 100 | 94 | 92 | 100 | 100 | 99 | 96 | 96 | 67 | 67 | 62 | 67 | 67 | 67 | 17 | - | - | - |

**FIG. 3**

○ SELECT DAY OF WEEK

| MON. | TUE. | WED. | THUR. | FRI. |
|------|------|------|-------|------|

○ SELECT PERIOD

| THIS WEEK | ~ 1 MONTH | ~ 3 MONTHS | ~ 12 MONTHS |
|-----------|-----------|------------|-------------|

○ SELECT TIME

| 7am~10pm | 24H |
|----------|-----|

|     | 12am | 1am | 2am | 3am | 4am | 5am | 6am | 7am | 8am | 9am | 10am | 11am | 12pm | 1pm | 2pm | 3pm | 4pm | 5pm | 6pm | 7pm | 8pm | 9pm | 10pm | 11pm |
|-----|------|-----|-----|-----|-----|-----|-----|-----|-----|-----|------|------|------|-----|-----|-----|-----|-----|-----|-----|-----|-----|------|------|
| A01 | -    | -   | -   | -   | -   | -   | -   | -   | 77  | 91  | 82   | 96   | 76   | 92  | 78  | 88  | 58  | 20  | 13  | 11  | 7   | 2   | -    | -    |
| A02 | -    | -   | -   | -   | -   | -   | -   | -   | 87  | 100 | 90   | 88   | 90   | 87  | 89  | 97  | 86  | 43  | 22  | 8   | 3   | -   | -    | -    |
| A03 | -    | -   | -   | -   | -   | -   | -   | -   | 88  | 79  | 93   | 91   | 83   | 86  | 75  | 77  | 66  | 36  | 21  | 11  | 1   | 8   | 6    | -    |
| A04 | -    | -   | -   | -   | -   | -   | -   | -   | 63  | 100 | 88   | 70   | 77   | 95  | 87  | 86  | 71  | 51  | 23  | 14  | 8   | 1   | 6    | 1    |
| A05 | -    | -   | -   | -   | -   | -   | -   | -   | 78  | 74  | 69   | 77   | 77   | 79  | 82  | 61  | 62  | 41  | 21  | 21  | 13  | 6   | -    | -    |

○ SELECT DAY OF WEEK

| MON. | TUE. | WED. | THUR. | FRI. |

○ SELECT PERIOD

| THIS WEEK | ~ 1 MONTH | ~ 3 MONTHS | ~ 12 MONTHS |

○ SELECT TIME

| 7am~10pm | 24H |

| MORNING UTILIZATION RATE | AFTERNOON UTILIZATION RATE | | 07:00 | 07:30 | 08:00 | 08:30 | 09:00 | 09:30 | 10:00 | 10:30 | 11:00 | 11:30 | 12:00 | 12:30 | 13:00 | 13:30 | 14:00 | 14:30 | 15:00 | 15:30 | 16:00 | 16:30 | 17:00 | 17:30 | 18:00 | 18:30 | 19:00 | 19:30 | 20:00 | 20:30 | 21:00 | 21:30 |
|---|---|---|---|---|---|---|---|---|---|---|---|---|---|---|---|---|---|---|---|---|---|---|---|---|---|---|---|---|---|---|---|---|---|
| 93.9 | 88.8 | S01 | - | - | 24 | 75 | 92 | 94 | 93 | 93 | 93 | 91 | 95 | 91 | 92 | 93 | 94 | 93 | 90 | 90 | 86 | 77 | 69 | 53 | 38 | 27 | 15 | 11 | 8 | 7 | 6 | 2 |
| 83.4 | 45.9 | S02 | - | - | 15 | 65 | 83 | 88 | 87 | 84 | 88 | 81 | 79 | 75 | 73 | 64 | 63 | 57 | 55 | 39 | 23 | 16 | 8 | 7 | 2 | 2 | 1 | - | - | - | - | - |
| 79 | 85.9 | S03 | - | - | 17 | 67 | 88 | 86 | 85 | 88 | 80 | 67 | 60 | 69 | 86 | 90 | 92 | 92 | 89 | 80 | 84 | 82 | 65 | 43 | 30 | 22 | 15 | 9 | 8 | 6 | 4 | 4 |
| 82.7 | 74.2 | S04 | - | - | 18 | 59 | 89 | 88 | 76 | 84 | 88 | 87 | 79 | 72 | 78 | 80 | 76 | 77 | 78 | 74 | 72 | 59 | 61 | 43 | 27 | 17 | 15 | 11 | 6 | 6 | 4 | 6 |
| 92.8 | 75.3 | S05 | - | - | 25 | 76 | 93 | 95 | 94 | 93 | 92 | 89 | 88 | 90 | 81 | 81 | 80 | 75 | 73 | 71 | 71 | 69 | 55 | 51 | 42 | 31 | 24 | 16 | 11 | 9 | 10 | 10 |

⋮

| NUMBER OF OPERATING ROOMS (AVERAGE) | | | | | |
|---|---|---|---|---|---|
| 16:00 | 16:30 | 17:00 | 17:30 | 18:00 | 18:30 |
| 8 | 8 | 7 | 6 | 4 | 3 |

─●─ NUMBER OF OPERATING ROOMS WITH UTILIZATION RATE EXCEEDING 50 %

─○─ NUMBER OF OPERATING ROOMS WITH UTILIZATION RATE BELOW 90 %

FIG. 4

# FIG. 5

START

OBTAIN INFORMATION ABOUT PATIENT'S ENTRY AND EXIT TIMES IN OPERATING ROOM — S1010

CALCULATE UTILIZATION RATE OF OPERATING ROOM FOR EACH CERTAIN TIME PERIOD BASED ON ENTRY AND EXIT TIMES — S1020

RECEIVE CERTAIN TIME INFORMATION AND CALCULATE AVERAGE UTILIZATION RATE BY TIME PERIOD IN RECEIVED TIME INFORMATION — S1030

ALLOCATE OPERATING ROOM WHERE PATIENT CAN BE OPERATED ON BASED ON AVERAGE UTILIZATION RATE — S1040

END

**REFERENCES CITED IN THE DESCRIPTION**

**Patent documents cited in the description**

- CA 3161894 A1
- US 11341462 B1